# EUROPEAN PATENT APPLICATION

(11) **EP 2 055 277 A1**
(43) Date of publication of application: **06.05.2009**
(21) Application number: 09151812.6
(22) Date of filing: 07.12.2005
(51) Int. Cl.: A61F 11/08

(54) **Hearing protection device with acoustic filter element and method for manufacturing the same**

(62) Divisional of application: 05026730.1
(71) Applicant: Phonak AG, 8712 Staefa (CH)
(72) Inventor: Oberdanner, Hannes, 8633, Wolfhausen (CH)
(74) Representative: Schwan - Schwan - Schorer

(57) **Abstract**

The invention relates to a hearing protection device (10) comprising a shell to be worn at least partially within a user's ear canal (14), wherein the shell is provided with an acoustic filter element (24) for attenuating sound comprising open-pore porous material (28), wherein the shell is designed such that the acoustic filter element is open towards ambience and towards the user's ear canal, and wherein the acoustic filter element is integral with the shell.

## Description

The present invention relates to a hearing protection device comprising a shell to be worn at least partially within a user's ear canal and an acoustic filter element for attenuating sound and to a method for manufacturing such hearing protection device.

Generally, hearing protection devices may have a generic shape and be made of a soft material for adapting to the individual shape of the user's outer ear and ear canal or they may have a customized shape which is individually adapted to the shape of the user's outer ear and ear canal in the manufacturing process by measuring the shape of the user's outer ear and ear canal prior to manufacturing. In the latter case, the hearing protection devices may be made of a relatively hard material.

In order to be able to control the acoustic attenuation attained by the hearing protection device at least to some extent, hearing protection devices frequently include a passive acoustic filter. Such acoustic filter serves to reduce the maximum attainable acoustic attenuation in a defined manner by providing an acoustic by-pass to the shell or body of the hearing protection device.

One type of acoustic filters is known as tube filters or vent filters and essentially consists of a narrow tube which is designed such that the filter has a cut-off frequency below which the acoustic attenuation usually has a steep linear decrease. While with tube filters good variation of the average attenuation is obtainable, they inherently have much lower attenuation at low frequencies compared to higher frequencies. Further, in total, attenuation of tube filters is significantly non-linear over the audible frequency range. An example of tube filters can be found in US 5,832,094.

Another type of acoustic filters is known as membrane filters which comprise at least one sheet-like membrane placed within a sound channel. Membrane filters usually have a more linear attenuation than tube filters, but the achievable variability of the average attenuation (i.e. the attenuation averaged over the audible frequency range) is usually much lower than for tube filters. In particular, the practically available materials are not able to achieve high average attenuation. Further, membrane filters are difficult to manufacture. An example of membrane filters can be found in WO 01/76520 A1.

US 5,799,658 relates to a hearing protection earplug comprising a body made of a foam material into which a shank made of a porous material is inserted which projects beyond the outer end of the foam body in order to provide for a handle for handling the earplug manually. However, the shank made of porous material does not penetrate through the inner end of the body made of foam material. It is also mentioned in US 5,799,658 that the porous shank may serve as an embedded acoustic filter.

DE 41 19 615 A1 relates to an earplug comprising a shell forming a hollow chamber which is filled with a particulate material serving for sound attenuation. At the outer end and at the inner end the shell is provided with a sound opening.

DE 1 096 545 relates to an earplug comprising a closed shell which is filled with balls.

US 2,785,675 relates to an earplug comprising a hollow shell having sound openings at its inner end and at its outer end, which is filled with a cellular fibrous material such as milk weed floss.

WO 97/18779 A1 relates to a hearing protection eaplug comprising an acoustic filter element which comprises a porous ceramic material, wherein the acoustic filter element acts as a pressure regulator with a slow leak rate. The pressure regulator is located within a silicone tubing which is connected via an adapter and a plastic cone to the shell of the earplug.

FR 2.095.127 relates to a hearing protection earplug comprising a shell having a cavity filled with a filling material which is permeable to air. The cavity is closed by an insert comprising an opening. The sound attenuation provided by the earplug may be adjusted according to the user's needs by reducing or increasing the amount of filling material in the cavity.

It is an object of the invention to provide for a hearing protection device having an acoustic filter element, wherein both relatively linear attenuation and relatively high variability of the average attenuation can be achieved. It is a still further object of the invention to provide a hearing protection device with such a filter element in a particularly simple manner.

These objects are achieved by a hearing protection device as defined in claim 1 and by a corresponding manufacturing method as defined in claim 10.

In general, the invention is beneficial in that, by providing the acoustic filter element with an open-pore porous material, a more linear attenuation than with tube filters can be achieved, while higher variability of the average attenuation than with membrane filters can be achieved. In addition, better control of the average attenuation and hence less variance of the attenuation can be achieved than with membrane filters. In addition, manufacturing is easier and more reproducible than with membrane filters. The invention is particularly beneficial in that, by integrating the acoustic filter element into the shell during manufacturing of the shell, particularly easy and efficient manufacturing of the hearing protection device is achieved.

In an open-pore porous filter sound energy is dissipated by friction of the air molecules moving with a certain velocity within the pores. The pores have to be open, because otherwise the sound energy could not penetrate into the porous material. The porous material may have a pore size of from 1 to 500 µm, a porosity of from 0.05 to 0.90 (the porosity is defined as the ratio of the open air volume to the total volume), a structural index of from 1 to 12 (the structural index is defined as the ratio of the open air volume to the effective porous volume), and a flow resistance per unit length of from 0.1 to 1000 Pa s/m² (the flow resistance per unit length is defined as the ratio of the specific flow resistance to the layer thickness in the flow direction, with the specific flow resistance being defined as the ratio of the pressure difference across the material to the velocity of the air flow).

The acoustic filter element may comprise a first portion and a second portion which differ from each other regarding structure, in particular, regarding pore size and/or grain size.

The acoustic filter element may have a tube-like shape, and it may comprise at least one channel extending from the outer end to the inner end of the acoustic filter element.

The shell may be produced by an additive layer-by-layer build-up process, such as selective laser sintering of a powder material, for example, a polyamide powder material, whereby the outer shape of the shell is designed according to the previously measured inner shape of the user's outer ear and ear canal. An overview over additive layer-by-layer build-up processes is given, for example, in US 6,533,062 B1 or US 2003/0233583 A1. Such processes are also called "rapid prototyping".

Since the acoustic filter element is formed integral with the shell, the acoustic filter element likewise can be produced by the same additive layer-by-layer build-up process. The porous structure of the acoustic filter element in the case of selective laser sintering of a powder material can be achieved by using a lower total laser energy per unit volume of the powder material when sintering the porous material of the filter element compared to when sintering the remainder of the shell. Thereby it can be achieved that the grains of the powder material do not completely melt but rather melt only at the surface so that in the sintered material the grain structure is maintained to some extent, whereby open pores are created. As an alternative, the structure of the porous material of the filter element integral with the shell may be produced having a pre-defined geometry. In this case it may be preferable to use a three-dimensional printing, i.e. "ink-jet"-like technology (see, for example, US 6,569,373 B2) rather than selective laser sintering.

In the following, examples of the invention will be illustrated by reference to the attached drawings.

Fig. 1 is a schematic cross-sectional view of a first n example of a hearing protection device, which is not covered by the invention, as worn by a user;

Fig. 2 is a schematic cross-sectional view of a second example of a hearing protection which is not covered by the invention;

Fig. 3 is a view like Fig. 2, with a third example, which is not covered by the invention, being shown; and

Fig. 4 is a view like Fig. 2, with an embodiment of the invention being shown.

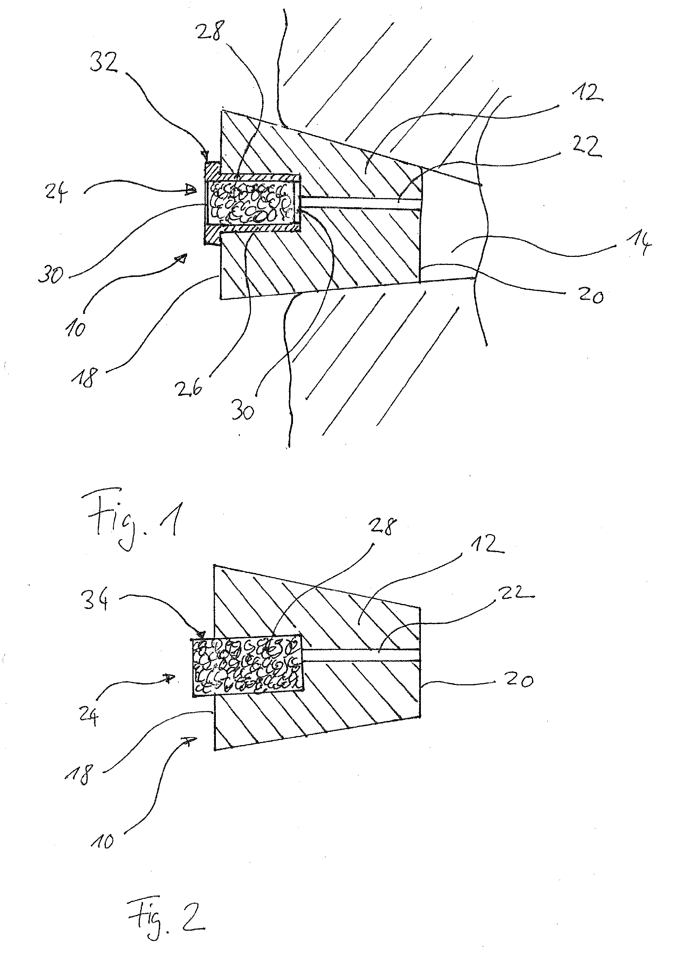

Fig. 1 is a schematic cross-sectional view of a hearing protection earplug 10, which is not covered by the invention and which comprises a shell 12 which is to be inserted into the user's ear canal 14 at least partially. Preferably the shell 12 is customized, i.e. it has an outer shape individually defined according to the measured inner shape of the user's outer ear and ear canal 14 (which can be measured, for example, by direct laser scanning of the ear or by laser scanning of an impression of the ear). The shell 12 may be produced according to the obtained individual ear shape data by an additive layer-by-layer build-up process, such as laser sintering of a powder material, such as a polyamide powder material. Alternatively, the layer-by-layer build-up process may be a three-dimensional printing, i.e. "ink-jet"-like process.

The shell 12 is formed with a receptacle 16 which is open towards the outer end 18 of the shell 12 and towards the inner end 20 of the shell 12, and hence the ear canal 14, via a sound passage 22.

The earplug 10 further comprises an acoustic filter element 24 for attenuating sound which is engaged with the receptacle 16 in a detachable manner. In the embodiment shown in Fig. 1, the filter element 24 completely fills the receptacle 16, with the inner end of the filter element 24 extending to the sound passage 22 and the outer end of the filter element 24 projecting outwardly beyond the outer end 18 of the shell 12 towards the ambience.

The filter element 24 comprises a housing 26 containing open-pore porous material 28. The housing 26 could be made, for example, of a plastics material or a metal or alloy. The housing 26 may have a tube-like shape and may have an inner and an outer open end which both are provided with an air-permeable membrane 30 for protecting the porous material 28. The porous material 28 may comprise a foamed material, sintered material, a fibre material, a particulate material, a fleece material, a braided material or a woven material and may be made of plastics, ceramics, metals, alloys and/or glass. Examples of such materials are foamed plastics, sintered polymers, foamed ceramics, sintered glass, sintered metal, mineral wool, rock wool, glass wool, kaolin wool, cotton, polycarbonate fibre, basalt wool, aluminium wool or wood fibre; woven material, braided material or fleece material made of metal, ceramic, synthetic fibres or natural fibres; sintered brass, cork, thermoplastic elastomeric materials, and/or polyurethane.

The pore size preferably is from 1 to 500 µm, the porosity is preferably from 0.05 to 0.90, the structural index preferably is from 1 to 12 and the flow resistance per unit length preferably is from 0.1 to 1000 Pa s/m².

The porous material 28 may be homogenous or it may be inhomogeneous in that it may comprise a plurality of different portions which differ from each other regarding structure, such as pore size and/or grain size, or regarding the material as such.

The parameters and the material/composition of the porous material 28, as well as the spatial arrangement thereof, can be used to tailor the desired acoustic attenuation provided by the filter element 24.

The filter element 24 is provided with an outwardly projecting portion 32 which can be manually operated in order to remove the filter element 24 from the shell 12 for replacement by an acoustic filter element of the same type or of a different type with different attenuation characteristic. The releasable engagement of the filter element 24 with the receptacle 16 can be realized, for example, by a bayonet-like connection, a snap-in connection, or an interference fit. Alternatively, the releasable engagement could be achieved by a relatively weak adhesive connection. In this case, the adhesive would have to be replaced when replacing the acoustic filter element 24.

Fig. 2 shows a modification of the embodiment of Fig. 1, which is not covered by the invention, wherein the housing 26 of the acoustic filter element 24 has been omitted so that the open-pore porous material 28 is directly engaged within the receptacle 16, for example, by elastic deformation of the porous material 28 or by an adhesive material. The porous material 28 is provided with an outwardly projecting portion 34 which enables the porous material 28 being manually removed from the receptacle 16 for replacement.

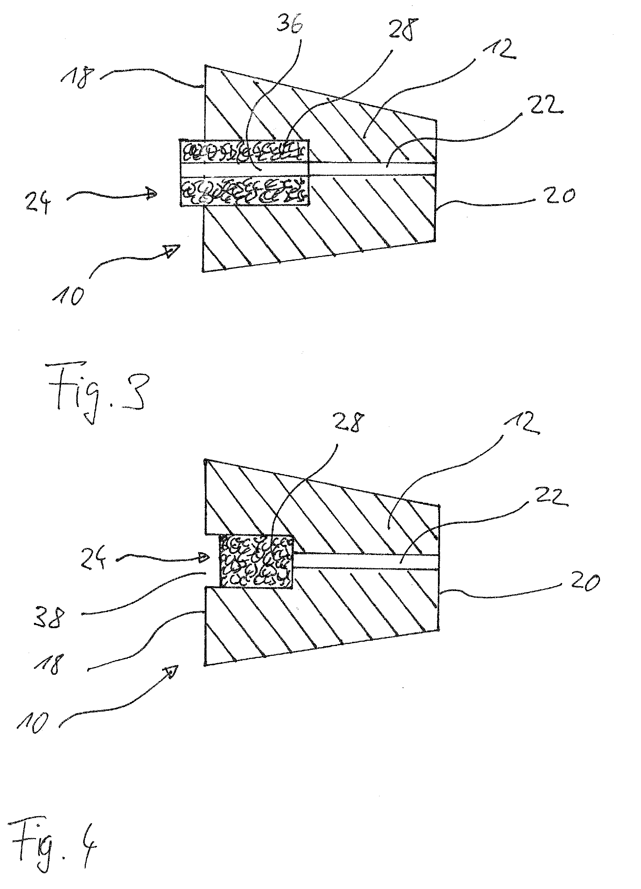

Fig. 3 shows a modified embodiment of Fig. 2, which is not covered by the invention, wherein the acoustic filter element 24, and hence the porous material 28, is provided with at least one channel 36 extending from the outer end to the inner end of the filter element 24. In this case, the filter element 24 may look like a hollow tube.

Fig. 4 shows an embodiment according to the invention, wherein the acoustic filter element 24 has been formed integral with the shell 12 during manufacturing of the shell 12. In this case the filter element 24 likewise has an open-pore porous structure and extends between the sound passage 22 and an outer opening 38 of the shell 12. If the shell 12 is produced by laser sintering of a powder material, the porous structure of the acoustic filter element 24 can be produced by reducing the total laser energy provided per unit volume compared to the production of the remainder of the shell 12. Thereby the powder particles would not melt completely, thereby leaving an interconnected void volume between the sintered particles of the powder material.

In an alternative approach, the open-pore porous structure of the filter element 24 could be realized with a pre-defined geometry. In this case, three-dimensional printing, i.e. "ink-jet"-like processes would be the most appropriate ones.

## Claims

1. Hearing protection device (10) comprising a shell (12) to be worn at least partially within a user's ear canal (14), wherein said shell is provided with an acoustic filter element (24) for attenuating sound comprising open-pore porous material (28), wherein the shell is designed such that the acoustic filter element is open towards ambience and towards the user's ear canal, and wherein the acoustic filter element is integral with the shell.

2. The device of claim 1, wherein the acoustic filter element (24) is acoustically connected to the ambience via an outer opening (38) of the shell (12) and is acoustically connected to the ear canal (14) via an inner opening (22) of the shell.

3. The device of one of claims 1 and 2, wherein the acoustic filter element (24) comprises a first portion and a second portion which differ from each other regarding pore size and/or grain size.

4. The device of one of the preceding claims, wherein the acoustic filter element (24) has a tube-like shape.

5. The device of claim 4, wherein the acoustic filter element (24) is provided with at least one channel (36) extending from the outer end to the inner end of the acoustic filter element.

6. The device of one of the preceding claims, wherein the porous material (28) has a pore size from 1 to 500 µm.

7. The device of one of the preceding claims, wherein the porous material (28) has a porosity from 0.05 to 0.90.

8. The device of one of the preceding claims, wherein the porous material (28) has a structural index from 1 to 12.

9. The device of one of the preceding claims, wherein the porous material (28) has a flow resistance per unit length of 0.1 to 1000 Pa·s/m².

10. Method for manufacturing a hearing protection device (10), comprising: producing a shell (12) adapted to be worn at least partially within a user's ear canal (14), wherein said shell is provided integrally with an acoustic filter element (24) for attenuating sound comprising open-pore porous material (28), and wherein the shell is designed such that the acoustic filter element is open towards ambience and towards the user's ear canal.

11. The method of claim 10, wherein the shell (12) including the acoustic filter element (24) is produced by an additive layer-by-layer build-up process.

12. The method of claim 11, wherein the shell (12) including the acoustic filter element (24) is made by laser sintering of a powder material.

13. The method of claim 12, wherein the total energy provided by the laser per unit volume of the powder material is lower when sintering the porous material (28) of the filter element (24) compared to when sintering the remainder of the shell (12).

14. The method of claim 11, wherein the structure of the porous material (28) of the filter element (24) is produced having a pre-defined geometry.

15. The method of one of claims 10 to 14, wherein the shell (12) is provided with an outer shape according to the measured inner shape of the user's outer ear and ear canal (14).
